# EUROPEAN PATENT SPECIFICATION

(11) **EP 0 669 802 B1**
(45) Date of publication and mention of the grant of the patent: **15.01.1997**
(21) Application number: 94900125.9
(22) Date of filing: 10.11.1993
(51) Int. Cl.: A01N 25/28, A01N 25/34, A01M 29/00

(54) **DEVICE FOR DEFENCE AGAINST INSECTS**
VORRICHTUNG ZUM SCHUTZ VON INSEKTEN
DISPOSITIF DE DEFENSE CONTRE LES INSECTES

(30) Priority: 17.11.1992 IT MI922620
(43) Date of publication of application: 06.09.1995
(73) Proprietor: LARUS PHARMA S.R.L., 20154 Milano (IT)
(72) Inventor: MODANESI, Bruno, I-20154 Milan (IT); BINI, Stefano, I-20121 Milan (IT)
(74) Representative: Marietti, Giuseppe
(86) International application number: EP9303154
(87) International publication number: WO9410842

(56) References cited:
- EP-A- 0 188 883
- EP-A- 0 348 550
- GB-A- 2 198 062
- US-A- 4 308 165
- Patent Abstracts of Japan, Vol 12, No 219, C-506, abstract of JP, 63-17803 (NIPPON FOIL MFG CO LTD et al.) 25 January 1988

## Description

### Technical Field

The present invention relates to a device for personal defence against insect attacks.

### Background Art

Devices and methods to keep mosquitoes and other insects at a distance from people and areas have been known for some time. Such devices, apart from physical screening by mosquito-net or similar means, usually repel the insects by means of smell or sound.

The most effective of these have been found to be those which use smell. It is known that it is mainly smell which attracts insects to people. In particular, mosquitoes are drawn by emissions of carbonic anhydride and water-vapour.

The first type of known repellent device releases substances into the atmosphere which are toxic or unpleasant to the insects - e.g. piretro and its derivatives - by combustion or vapourization. The main drawback of such devices is that they are in general unpleasant and in some cases even toxic, though to a moderate degree, to people in the environment. A further disadvantage is that such substances are effective in closed spaces, while outdoors or in open spaces they are only partially effective or effective in the immediate vicinity of the dispenser, with the obvious disadvantages that this entails.

A partial solution to these drawbacks are devices which consist of repellent lotions or creams which are applied directly to the body. These devices have the drawback that the repellent action of the composition, once applied, is comparatively short-lived, and the chemical substances which form the repellent composition come into direct contact with the skin with possible toxic or allergic effects. A further disadvantage is that the repellent composition must be applied to all of the exposed or lightly protected skin area.

GB-A-2198062 discloses packaging material in which microencapsulated materials such as animal repellant are applied as micro capsules to the surface of said packaging material, in a manner that the micro capsules can be broken during use of the material. The microcapsules can also be located within two superimposed portions of the package in an adhesive medium: when the portions are separated, they break at least some of the micro capsules.

### Disclosure of the invention

The aim of the present invention is to overcome the above mentioned disadvantages by means of a device which ensures safe and long-lasting insect repulsion without any contact between the skin and the repelling composition.

This aim is achieved by the present invention, which concerns an insect-repelling device, of a type containing one or more insect-repelling active substances, characterized by consisting of a container at least in part gas-permeable, and a plurality of pressure-rupturing microcapsules containing the said active composition within the said container.

According to a preferred embodiment, the container has a layer of material impermeable to gas and liquids with an external means of self-adhesion. This layer is coupled by a known method way to a second layer which is permeable to gas and liquids so as to permit the passage of vapours of the active substance of the repellent composition, once the said vapours have been liberated by crushing the microcapsules.

In this way the repellent composition remains within the microcapsules until it is used, and direct contact between the skin and the composition itself can be avoided.

According to another embodiment, the impermeable layer is coupled with a sponge-like material and this material is coupled with said second, permeable, layer.

### Best mode for carrying out the invention

A further advantage is that release of the composition can be controlled by gradually rupturing the microcapsules by pressure; in other words, the user can initially rupture only part of the capsules and then the remainder can be burst once when the initial repellent effect has subsided.

The invention will now be described in detail with reference to the enclosed drawings which are of an illustrative rather than limiting nature, in which :
- Fig. 1 is a transverse section of a device according to the invention;
- Figs. 2 and 3 are prospective views of two other embodiments of the device according to the invention;
- Fig. 4 is a sectional view of another preferred embodiment of the container; and
- Fig. 5 is a perspective view of a plurality of the containers of fig. 4 in a ready-for-sale condition.

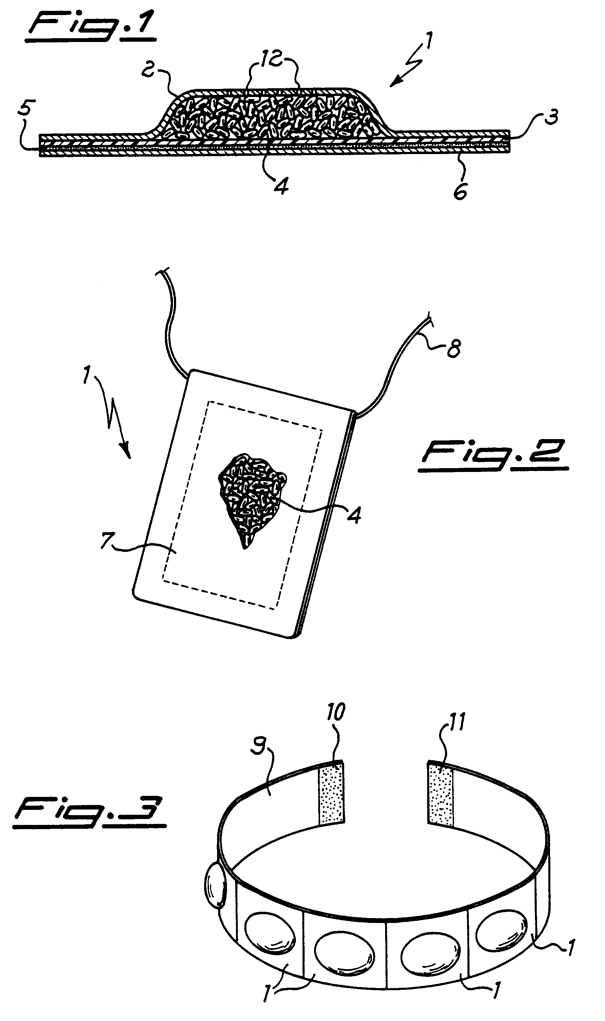

As already stated, the device according to the invention consists of a container 1 which in fig 1 consists of a layer of gas-permeable material 2 attached by a known method, e.g. adhesives or thermo-welding, to a layer of material 3 which is impermeable to gas and liquids and is generally made of a plastic material.

To increase the permeability to gas, the layer 2 may contain a plurality of holes 12.

According to the invention, a plurality of microcapsules 4 containing a repellent composition consisting of one or more insect-repelling active substances is contained between the two layers of material. The capsules are produced by a known method, e.g. in gelatine, or from cellulose derivatives such as sodium carboxymethylcellulose or similar materials used for this purpose and known in the art for this; the main requirement of the capsule is that it should crush-rupture under light pressure such as the user's finger-pressure.

To use the device according to the invention the user bursts some or all of the microcapsules to release the repellent composition contained within them.

Preferentially, none of the composition should come into contact with the skin or clothes of the user, because the impermeable material 3 prevents diffusion towards the user. This aspect of the invention is important in the case of compositions containing chemical compounds which might cause allergic reactions in particularly sensitive subjects.

Below the layer of material 3 is a layer of self-adhesive material 5 of the re-usable type, so that the device can be removed and re-attached to the user (either to skin or clothes), or in the vicinity of the user (to a table or bedhead). The adhesive layer 5 is protected by a layer of siliconized paper 6, which is removed just prior to use.

As an alternative to the embodiment described above, layers 2 and 3 may be made of the same or of similar material. For example, both may be made of absorbent material, such as a suitable type of paper or non-woven fabric, with the advantage of being able to regulate the release of the vapour of the active substance. In this embodiment, the re-usable adhesive layer 5 provides the required impermeable barrier of layer 3 of the container.

Fig.2 shows another embodiment of the device according to the invention.

According to this embodiment, the container 1 consists of a sachet 7 containing the microcapsules 4 and provided with strings 8 or similar means for attachment to the person or in the vicinity.

This embodiment allows the adhesive layer 5 to be dispensed with.

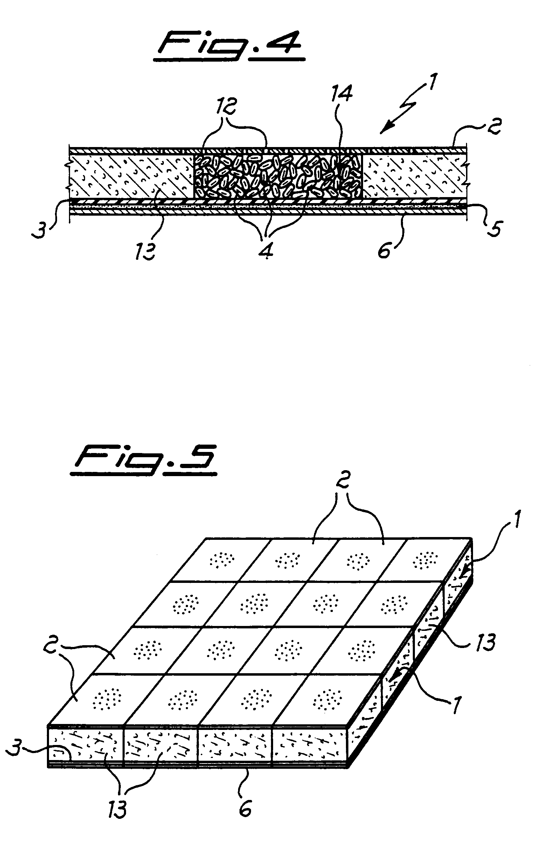

Fig.3 shows another embodiment of the device according to the invention, where a plurality of containers 1 according to Fig.1 are arranged in line on adhesive material 5 along a single strip of siliconized paper 9 which functions as the layer 6 in Fig.1.

The strip 9 has adhesive ends 10 and 11 which allow the strip to be closed on itself and worn as a bracelet, from which the individual containers 1 can be removed, if desired, and attached to the required place. Alternatively the containers can be burst directly on the bracelet 9.

In the preferred embodiment disclosed in fig. 4, container 1 is still provided with the same layers 2 to 6 of the embodiment of fig. 1, but in this case upper layer 2 is coupled to element 13, made of a sponge-like porous material, that is also permanently adhered to layer 3. The material of element 13 is advantageously made of expanded plastic material, such as expanded polyurethane or polyethylene, known in the art and commercially available.

The thickness of element 13 is greater than that of layers 2 and 3, and element 13 is provided with a hole 14 that serves as a container for microcapsules 4.

The advantage of this embodiment with respect to that of fig. 1, is that it is much easier to be manufactured. This is done by first punching a plurality of holes in a sheet of expanded plastic material and by successively filling the punched holes 14 with capsules 4, coupling layers 3 and 2 at opposite sides of said sheet, and finally punching sheet and layers to obtain a plurality of containers 1. Most preferably, said containers are not completely separated from each other, as shown in fig. 5, to provide a small "sheet" of containers ready for sale.

The active substance in the capsule can be chosen from any known insect repellent and is usually selected from the following compounds: basil essential oil, citronella essential oil, geranium essential oil, lavender essential oil, rhodinol, geraniol, citronellol, citral, benzil, benzylbenzoate, N-butylacetanilide, Indalone (TM of FMC Corp., USA) , N,N-diethyl-m-toluamide, dimethylcarbate, derivatives of cyclohexanol, 2-ethyl-2-butyl-1,3-propandiol, 2,5-dimethyl-2,5-hexandiol and similar higher alcohols,
phthalates of the formula 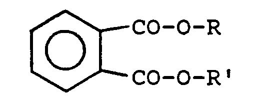 where R and R' are C1-C5 alkyles,
butylesters of bicarboxylic acid of the formula

H₉C₄-O-OC-(CH2)n-CO-O-C₄H₉

where n is an integer between 2 and 6,
and mixtures thereof.

In the container with the capsules 4 there may also be other substances such as inert absorbers or adsorbers to retain the composition released by the burst capsules and release it gradually into the environment. Such inert substances are preferably chosen from calcium sulphate, with its various gradations of hydration, silicates, calcium carbonate, crushed porous clays - both natural and expanded, zeolite and similar materials. The size of this material particles is preferably less than that of the microcapsules and its weight is between 10% and 45% of the capsules.

The invention will now be further described with references to the following examples of use:

### EXAMPLE 1

Three devices according to the present invention containing essence of citronella oil were applied to a male adult subject. The devices were located as follows:
one at the nape of the neck and one on each calf, leaving the head, arms and legs uncovered.

The subject was exposed in the open in a damp area after dusk in the month of August, for a period of 1.5 hours, unprotected in any other way from insects.

At the end of the period skin lesions due to insect bites were counted.

Then the previously applied devices were removed and same subject was exposed in identical conditions for a period of 15 minutes.

At the end of this second period skin lesions due to insect bites were counted.

### EXAMPLES 2 to 4

The procedure described in Example 1 was followed another three times with three different subjects using different active substances :
essence of basil oil for subject 2;
essence of lavender and citronella oils for subject 3;
essence of geranium and basil oils for subject 4.

The results of all four tests are shown below:

**TABLE 1**

| | Protected | Unprotected |
|---|---|---|
| Subject 1 | - | 5 |
| Subject 2 | - | 3 |
| Subject 3 | - | 7 |
| Subject 4 | - | 4 |

## Claims

1. Insect repellent device, of the type using a composition containing one or more insect repelling active substance in a plurality of pressure-rupture microcapsules (4), characterized in that it comprises a container (1) at least in part gas-permeable, containing the said active substance within the said microcapsules (4).

2. A device according to claim 1, wherein the container (1) is provided with a portion (3; 5) impermeable to gas and liquids.

3. A device according to claims 1 or 2, wherein the active substance in the capsules (4) is selected from the following compounds: basil essential oil, citronella essential oil, geranium essential oil, lavender essential oil, geraniol, citronellol, citral, benzil, benzylbenzoate, N-butylacetanylide, Indalone, N,N-diethyl-m-toluamide, dimethylcarbate, derivatives of cyclohexanol, 2-ethyl-2-butyl-1,3-propandiol, 2,5-dimethyl-2.5-hexandiol and similar higher alcohols, phthalates of the formula 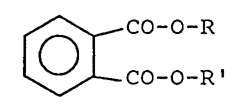 where R and R' are C1-C5 alkyles,
butylic esters of bicarboxylic acids of the formula :
H₉C₄-O-OC-(CH2)n-CO-O-C₄H₉
where n is an integer between 2 and 6,
and mixtures thereof.

4. A device according to claim 3, wherein the said active substance essentially consists of essential oils selected from : basil, geranium, citronella, lavender or a mixture thereof.

5. A device according to any preceding claim, wherein the container (1) further comprises one or more absorber or adsorber materials to slow the release into the ambient of the active substances.

6. A device according to claim 5, wherein said substances are inerts selected from calcium sulphate at any hydration degree, silicates, calcium carbonate, crushed porous clays, zeolites or a mixture thereof.

7. A device according to any previous claim, wherein at least part of the container is made of an absorbent material.

8. A device according to any previous claim, wherein the said container (1) has a first layer (3) of material impermeable to gas and liquids and fitted externally with self-adhesive means of attachment (5), said impermeable layer (3) being bound to a second layer (2), opposite the first, made of gas-permeable material, the said microcapsules (4) containing the repellent composition being contained between the said two layers (2,3).

9. A device according to any previous claim, wherein said first layer (3) and said second layer (3) are coupled to an intermediate element (13) of porous material having a cavity (14) housing said microcapsules (4).

10. The use of a device according to claim 8 for repelling insects.

## Patentansprüche

1. Insektenvertreibende Einrichtung unter Verwendung einer Zusammensetzung, die eine oder mehrere insektenvertreibende, aktive Substanzen in einer Vielzahl von unter Druck aufbrechenden Mikrokapseln (4) enthält, gekennzeichnet dadurch, daß sie ein mindestens teilweise gasdurchlässiges Behältnis (1) umfaßt, das die aktive Substanz in den Mikrokapseln (4) enthält.

2. Einrichtung nach Anspruch 1, bei der das Behältnis (1) mit einem Bereich (3; 5) versehen ist, der für Gas und Flüssigkeiten undurchlässig ist.

3. Vorrichtung nach Anspruch 1 oder 2, bei der die aktive Substanz in den Kapseln (4) aus den folgenden Verbindungen ausgewählt wird: ätherisches Basilienöl, ätherisches Zitronellöl, ätherisches Geraniumöl, ätherisches Lavendelöl, Geraniol, Zitronellol, Zitral, Benzil, Benzylbenzoat, N-Butylazetanilid, Indalone, N,N-Diethyl-m-Toluamid, Dimethylcarbat, Derivaten aus Cyklohexanol, 2-Ethyl-2-Butyl-Propan-1,3-diol, 2,5-Dimethylhexan-2,5-diol und ähnlichen höheren Alkoholen, Phthalaten von der Formel 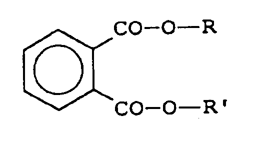 worin R und R' C1-C5-Alkyle sind,
Butylester aus Bikarbonsäuren von der Formel: H₉C₄O-OC-(CH2)n-CO-O-C₄H₉, worin n eine ganze Zahl zwischen 2 und 6 ist,
und Mischungen daraus.

4. Vorrichtung nach Anspruch 3, bei der die aktive Substanz im wesentlichen aus ätherischen Ölen besteht, die ausgewählt werden aus: Basilikum, Geranie, Zitrone, Lavendel oder einer Mischung daraus.

5. Einrichtung nach einem beliebigen vorhergehenden Anspruch, bei der das Behältnis (1) außerdem einen oder mehrere Absorber- oder Adsorbermaterialien umfaßt, um das Freisetzen der aktiven Substanzen in die Umgebung zu verlangsamen.

6. Einrichtung nach Anspruch 5, bei der die Substanzen inerte Stoffe sind, die aus Kalziumsulfat eines beliebigen Hydratationsgrades, Silikaten, Kalziumkarbonat, zerkleinerten porösen Tonen, Zeolithen oder einer Mischung daraus ausgewählt wird.

7. Einrichtung nach einem beliebigen vorhergehenden Anspruch, bei der mindestens ein Teil des Behältnisses aus einem absorbierenden Material hergestellt ist.

8. Einrichtung nach einem beliebigen vorhergehenden Anspruch, bei der das Behältnis (1) eine erste Schicht (3) aus einem Material hat, das für Gas und Flüssigkeiten undurchlässig ist und extern mit selbsthaftenden Befestigungsmitteln (5) befestigt ist, wobei die undurchlässige Schicht (3) an einer zweiten, aus einem gasdurchlässigen Material hergestellten Schicht (2) gegenüber der ersten befestigt ist und die Mikrokapseln (4), die die Repellentzusammensetzung enthalten, zwischen den beiden Schichten (2, 3) aufgenommen sind.

9. Einrichtung nach einem beliebigen vorhergehenden Anspruch, bei der die erste Schicht (3) und die zweite Schicht (3) mit einem Zwischenelement (13) aus porösem Material verbunden sind, das einen Hohlraum (14) besitzt, der die Mikrokapseln (4) aufnimmt.

10. Anwendung einer Einrichtung gemäß Anspruch 8 zur Vertreibung von Insekten.

## Revendications

1. Dispositif repoussant les insectes, du type utilisant une composition contenant une ou plusieurs substances répulsives pour les insectes dans un grand nombre de microcapsules (4) qui se brisent sous l'effet d'une pression, caractérisé en ce qu'il comprend un récipient (1) au moins en partie perméable aux gaz contenant cette substance active à l'intérieur de ces microcapsules (4).

2. Dispositif suivant la revendication 1, dans lequel le récipient (1) est pourvu d'une partie (3, 5) imperméable aux gaz et aux liquides.

3. Dispositif suivant les revendications 1 ou 2, dans lequel la substance active présente dans les capsules (4) est choisie parmi les composés suivants : huile essentielle de basilic, huile essentielle de citronelle, huile essentielle de géranium, huile essentielle de lavande, géraniol, citronellol, citral, benzile, benzoate de benzyle, N-butylacétanilide, Indalone, N,N-diéthyl-m-toluamide, diméthyl carbate, dérivés de cyclohexanol, 2-éthyl-2-butyl-1,3-propanediol, 2,5-diméthyl-2,5-hexanediol et alcools supérieurs analogues, phtalates de formule 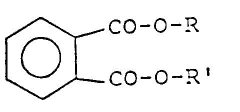 dans laquelle R et R' sont des groupes alkyles en C₁₋₅, esters butyliques d'acide dicarboxylique de formule :
H₉C₄-O-OC-(CH₂)ₙ-CO-O-C₄H₉
dans laquelle n est un entier compris entre 2 et 6, et leurs mélanges.

4. Dispositif suivant la revendication 3, dans lequel cette substance active consiste essentiellement en huiles essentielles choisies parmi le basilic, le géranium, la citronelle, la lavande ou leur mélange.

5. Dispositif suivant l'une quelconque des revendications précédentes, dans lequel le récipient (1) comprend de plus, une ou plusieurs matières absorbantes ou adsorbantes pour ralentir la libération dans l'air ambiant des substances actives.

6. Dispositif suivant la revendication 5, dans lequel ces substances sont des matières inertes choisies parmi un sulfate de calcium à un quelconque degré d'hydratation, des silicates, un carbonate de calcium, des argiles poreuses écrasées, des zéolites et leur mélange.

7. Dispositif suivant l'une quelconque des revendications précédentes, dans lequel au moins une partie du récipient est faite d'une matière absorbante.

8. Dispositif suivant l'une quelconque des revendications précédentes, dans lequel ce récipient (1) compren une première couche (3) de matière imperméable aux gaz et aux liquides et pourvue extérieurement d'un moyen auto-adhésif de fixation (5), cette couche imperméable (3) étant liée à une seconde couche (2), opposée de la première, faite d'une matière perméable aux gaz, ces microcapsules (4) contenant la composition répulsive étant contenues entre ces deux couches (2, 3).

9. Dispositif suivant l'une quelconque des revendications précédentes, dans lequel cette première couche (3) et cette seconde couche (2) sont couplées à un élément intermédiaire (13) de matière poreuse comprenant une cavité (14) pour loger ces microcapsules (4).

10. Utilisation d'un dispositif suivant la revendication 8, pour repousser les insectes.
